# EUROPEAN PATENT SPECIFICATION

(11) **EP 1 239 840 B1**
(45) Date of publication and mention of the grant of the patent: **06.04.2005**
(21) Application number: 00984181.8
(22) Date of filing: 08.12.2000
(51) Int. Cl.: A61K 9/22, A61P 31/18

(54) **ANTIVIRAL MEDICATION**
ANTIVIRALE ARZNEI
MEDICATION ANTIVIRALE

(30) Priority: 09.12.1999 US 169883 P
(43) Date of publication of application: 18.09.2002
(73) Proprietor: Alza Corporation, Mountain View, CA 94039-7210 (US)
(72) Inventor: DONG, Liang, C., Sunnyvale, CA 94086 (US); ESPINAL, Steven, D., Mountain View, CA 94043 (US); WONG, Patrick, S.-L., Burlingame, CA 94010 (US); MAGRUDER, Paul, R., Mountain View, CA 94040 (US)
(74) Representative: Williams, Paul Edwin
(86) International application number: PCT/US2000/033542
(87) International publication number: WO 2001/041742

(56) References cited:
- WO-A-00/13674
- WO-A-00/28942
- WO-A-00/35419
- WO-A-98/22106
- US-A- 5 324 280
- US-A- 5 358 721
- US-A- 5 830 502

## Description

This invention pertains to a dosage form.
More particularly, the invention pertains to a sustained release oral dosage form comprising a liquid antiviral drug formulation (also referred to as an antiviral pharmaceutical composition).

### Background of the Invention

A pressing need exists for antiviral medications. The need exists, for example, for antiviral medication for the management of the human immunodeficiency virus. The human immunodeficiency virus (HIV) is a retrovirus, which is the etiological agent for acquired immune deficiency syndrome, (AIDS). The virus is transmitted generally by parenteral inoculation, and by intimate social contact. A majority of those now infected with the virus will develop the acquired immune deficiency syndrome in a follow up period, usually seven to ten years.

Intensive research efforts to develop medications that can lessen or block the development of serious clinical symptoms in HIV-infected patients have lead to the discovery of protease inhibitors. Protease inhibitors effect the life cycle of viruses, thus, they are used as antiviral medications. Protease inhibitors operate by effecting the cleavage of high-molecular-weight viral polyprotein precursors necessary for the assembly and morphogenesis of viruses (Clin Neurobiol Rev Vol 11, p. 614-627, (1998)). Protease inhibitors, also known as antiretroviral drugs, such as saquinavir, ritonavir, indinavir, nelfinavir and amprenavir, are used to treat patients infected with the human immunodeficiency virus (Clin Ther, Vol 19, No. 2, p. 187-214, (1997)).

International Publication Number WO 00/35419 discloses a dosage form comprising a gelatin capsule containing a liquid, active agent formulation where the wall comprises a barrier layer formed over the external surface of the capsule, an expandable layer formed over the barrier layer, a semipermeable layer formed over the expandable layer, and an exit orifice through which the liquid, active agent formulation can be delivered to an environment of use.

International Publication Number WO 00/13674 discloses a dosage form comprising a semipermeable walled container that houses a capsule. In another embodiment, the capsule also includes a movable piston positioned between the drug formulation and the osmotic composition. The drug formulation comprises 0.5wt% to 65wt% of a drug and 0.5wt% to 60wt% of a pharmaceutically acceptable liquid carrier. The carrier may include a surfactant that reduces aggregation of components in the formulation and/or imparts emulsification to the formulation and an oil phase.

International Publication Number WO98/22106 discloses a liquid pharmaceutical composition for the administration of inhibitors of HIV protease. The composition is a solution comprising the inhibitor, a pharmaceutically acceptable organic solvent and, optionally, a pharmaceutically acceptable surfactant The solution may be encapsulated in a gelatin capsule.

US 5 358 721 discloses hydro-activated osmotic dosage forms for antiviral drugs comprising a semi-permeable wall, a fluid receiving internal compartment, exit means in the semi-permeable wall, a fluid-activated displacement layer in the compartment, and a composition layer comprising the antiviral drug and an antiviral drug flux promoter.

There are serious problems associated with the use of currently available protease inhibitors. For example, protease inhibitors exhibit a potential for forming drug resistance-HIV mutates so they may lose their potency in the absence of protection in an acceptable dosage form. Further, since many drugs require three or more doses a day, poor patient compliance can be a significant factor leading to drug resistance. The drug also can exhibit an intrinsic aggregation tendency at hydrated states, which dictates against incorporating the antiviral protease inhibitors into a controlled-sustained release dosage form. Additionally, protease inhibitors are solids with limited absorption and poor aqueous solubility. Thus, protease inhibitors have low bioavailability. These properties do not lend themselves for formulation into pharmaceutical compositions for delivery from dosage forms that are therapeutically acceptable.

Therefore, there is a need for dosage forms comprising antiviral drug formulations that deliver a therapeutically acceptable level of the antiviral drug at a controlled-sustained release rate to a patient in need of antiviral therapy. The demand exists for delivery means for gastrointestinal delivery of antiviral drug for preventing *in-situ* aggregation effects of the antiviral drug, and obtaining improved bioavailability of the antiviral drug, which is substantially independent of the variable environment of the gastrointestinal tract.

### Summary of the Invention

The present invention pertains to a sustained release oral dosage form comprising a liquid antiviral drug formulation (also referred to as an antiviral pharmaceutical composition), and is defined by the claims. A pharmaceutical composition comprising the liquid antiviral drug formulation, and a method of treatment comprising administering the sustained release oral dosage form comprising the antiviral pharmaceutical composition are also disclosed.

The inventors herein have found, surprisingly, that a pharmaceutical composition comprising a liquid antiviral drug formulation is substantially free of *in*-situ aggregation effects of the antiviral drug. Additionally, the inventors have discovered that the antiviral pharmaceutical composition has several advantages: (1) the antiviral pharmaceutical composition has a high loading of the antiviral drug, with up to 60 wt% of the antiviral drug dose solubilized in the composition; (2) the antiviral pharmaceutical composition has substantially improved bioavailability of the antiviral drug; and (3) controlled sustained release of high doses, up to 300 mg, of the antiviral drug substantially improves patient compliance and reduces the likelihood of drug resistance.

Antiviral drugs such as protease inhibitors are solids with limited absorption, poor aqueous solubility and low bioavailability. The antiviral pharmaceutical composition described herein provides a sustained release oral dosage form to deliver a therapeutically acceptable level of the antiviral drug to a patient in need thereof. Additionally, the antiviral pharmaceutical composition provides improved bioavailability of the antiviral drug and is substantially independent of the variable environment of the gastrointestinal tract.

A pharmaceutical composition comprising a liquid antiviral drug formulation may be used in the sustained release dosage form of the invention, wherein the pharmaceutical composition is substantially free of *in-situ* aggregation effect of the antiviral drug, and provides substantially improved bioavailability of the antiviral drug. The dosage form is adapted to administer a therapeutically effective dose of the antiviral drug over a period of at least 4 hours after oral administration. The pharmaceutical composition comprises an antiviral drug solubilized in a solvent, a hydrogel and optionally an osmagent.

The antiviral drug may be a protease inhibitor and the solvent may be a non-ionic surfactant, with up to 60wt% of the antiviral drug dose being dissolved in the pharmaceutical composition.

According to one aspect of the present invention there is provided a sustained release oral dosage form comprising:
(a) a wall defining a compartment, the wall comprising a semipermeable layer;
(b) an expandable layer located within the compartment and in fluid communication with the semipermeable layer;
(c) a capsule located within the compartment and in direct or indirect contacting relationship with the expandable layer, the capsule comprising a liquid antiviral drug composition comprising an antiviral drug solubilized in a solvent, which composition is substantially free of in-situ aggregation effect of the antiviral drug and provides substantially improved bioavailability of said antiviral drug; and
(d) an exit orifice formed or formable in the dosage form extending from the external surface of the capsule to the environment of use.
In the designated states of AT, BE, CH, DE, DK, ES, FR, GB, GR, IE, IT, LI, LU, MC, NL, PT, SE, FI and CY, the antiviral drug is also present in an amount of 5 wt% to 60 wt% and the solvent is present in an amount of 20 wt% to 95 wt% of the total antiviral drug composition. In the designated state of Turkey, these relative amounts of antiviral drug and solvent are optional.

In certain embodiments of the dosage form, the expandable layer may be located within the capsule and may be remote from the exit orifice, and a barrier layer may be located within the capsule between the antiviral pharmaceutical composition and the expandable layer. In alternative embodiments of the dosage form, the expandable layer may be located within the compartment between the capsule and the semipermeable layer, and a barrier layer may be located within the compartment between the capsule and the expandable layer.

The semipermeable layer may comprise a semipermeable polymer, and the expandable layer may comprise a hydrophilic polymer. The expandable layer may further comprise a lubricant and/or an osmotically effective compound.

In certain embodiments, the hydrophilic polymer is present in an amount of up to 95 wt%, the osmotically effective agent is present in an amount of 0 wt% to 60 wt%, and the lubricant is present in an amount of 0 wt% to 5 wt% of the total composition of the expandable layer.

The solvent may comprise a surfactant, an oil or mixtures thereof.

The surfactant may be a non-ionic surfactant.

In certain embodiments, the liquid antiviral drug composition may comprise a hydrogel and/or an osmagent.

The antiviral drug may be selected from the group consisting of acyclovir, azidouridine, anasmycin, amantadine, bromovinyldeoxusidine, chlorovinyldeoxusidine, cytarbine, didanosine, deoxynojirmycin, dideoxycitidine, dideoxyinosine, dideoxynudeoside, desciclovir, deoxyacyclovir, edoxuidine, enviroxime, fiacitabine, foscarnet, fialuridine, fluorothymidine, fluxuridine, ganciclovir, hypericin, interferon, interlenkin, isethionate, idoxuridine, nevirapine, pentamidine, ribavirin, rimantadine, stavirdine, sargramostin, suramin, trichosanthin, trifluorothymidine, tribromothymidine, trichlorothymidine, vidarabine, zidoviridine, zalcitabine, 3-azido-3-deoxythymidine, saquinavir, adefovir, ritonavir, indinavir, nelfinavir, amprenavir, zidovudine and zalcitabin. Preferably, the antiviral drug is a protease inhibitor.

Preferably, the capsule is a gelatin capsule.

Preferably, the dosage form can produce an average steady-state plasma concentration of the antiviral drug greater than the therapeutically effective concentration of the antiviral drug over a period of about 4 hours to about 24 hours.

In preferred embodiments, the dosage form is for use in treating a condition in a subject responsive to the antiviral drug, wherein said condition is acquired immune deficiency syndrome (AIDS) associated with human immunodeficiency virus (HIV) infection in the subject.

In other embodiments, the dosage form may administer a therapeutically effective dose of said antiviral drug over a period of at least 4 hours after administration with no more than 30% by weight of said drug composition being released within the first 1 hour after oral administration.

In other embodiments, the dosage form may administer a therapeutically effective dose of said antiviral drug over a period of at least 12 hours after administration with no more than 30% by weight of said drug composition being released within the first 4 hours after oral administration.

In other embodiments, the dosage form may administer a therapeutically effective dose of said antiviral drug over a period of 24 hours after administration with no more than 30% by weight of said drug composition being released within the first 12 hours after oral administration.

Also described (but not part of the invention) is a Method of treating a condition in a subject responsive to antiviral medication, the method comprising orally administering to the subject a sustained release dosage form comprising an antiviral pharmaceutical composition as described above.

These and other embodiments of the present invention will readily occur to those of ordinary skill in the art in view of the disclosure herein.

### Brief Description of the Figures

Figure 1 illustrates the release patterns for two delivery systems provided by the invention.
Figure 2 illustrates the release profile of a liquid delivery system, for the antiviral ritonavir.

### Detailed Description of the Invention

As used in this specification and the appended claims, the singular forms "a," "an" and "the" include plural references unless the content clearly dictates otherwise.

The term "aggregation effect of the antiviral drug" as used herein means the antiviral drug can cluster together but do not fuse. The terms "antiviral drug" and "antiviral medication" are used interchangeably herein. Aggregation is a scientific phenomenon that can lead to sedimentation that substantially prevents the absorption of the antiviral drug at a controlled-sustained rate over time. Sedimentation as used herein denotes the formation of an insoluble aggregation that may settle by gravitation. The pharmaceutical composition comprising a liquid antiviral drug formulation as described herein provides a composition substantially free of *in-situ* aggregation effect, thus substantially improving the bioavailability of the antiviral drug.

The term "surfactant" as used herein denotes a surface-active substance that reduces the surface tension of water. In preferred embodiments, the surfactant is a liquid non-ionic surfactant or an edible oil.

The terms "controlled release rate" and "controlled rate" are used interchangeably herein, and refer to the release or delivery of a drug, e.g., from a dosage form, at a constant rate for maintaining a constant drug level in blood-plasma or in a tissue. Sustained-release as used herein denotes the release of drug over an extended period of time, including the time of gastrointestinal transit.

The term "liquid antiviral drug formulation" as used herein refers to an antiviral drug solubilized in a solvent, the solvent preferably comprisingsurfactants, oils or mixtures thereof. The liquid formulation may exist as a self-emulsifying composition wherein the antiviral drug is solubilized or suspended in the surfactant or a lipophilic component, which when dispersed in an aqueous environment forms become micelles or an emulsion. The results of the liquid spreading over the surface of the drug avoids drug agglomeration, thus providing the pharmaceutical composition as described herein which is substantially free of *in-situ* aggregation effect of the antiviral drug.

The term "pharmaceutical composition comprising a liquid antiviral formulation"; "an antiviral drug composition"; "an antiviral pharmaceutical composition"; and "a pharmaceutical composition" are used interchangeably herein.

The terms "protease inhibiting" and "protease inhibitor" as used interchangeably herein, and refer generally to a drug that prevents the reproduction of the human immunodeficiency virus (HIV) in a patient. Protease inhibitors operate by blocking an enzyme of HIV called protease. With the protease blocked, the HIV virus can't mature to infect cells. Clinical studies of protease inhibitors have shown these drugs can lower the viral load and raise T-cells.

### I. Modes of Carrying Out the Invention

The present invention pertains to a sustained release oral dosage form comprising a liquid antiviral drug formulation (also referred to as an antiviral pharmaceutical composition), which is defined by the claims Also described is a pharmaceutical composition comprising the antiviral pharmaceutical composition; and a method of treatment (not part of the invention) comprising administering the sustained release oral dosage form comprising the antiviral composition. The inventors herein have found, surprisingly, that a pharmaceutical composition comprising a liquid antiviral drug formulation is substantially free of *in-situ* aggregation effects of the antiviral drug. Additionally, the inventors have discovered that the antiviral pharmaceutical composition has several advantages: (1) the antiviral pharmaceutical composition has a high loading of the antiviral drug, with up to 60 wt% of the antiviral drug dose solubilized in the composition; (2) the antiviral pharmaceutical composition provides substantially improved bioavailability of the antiviral drug; and (3) controlled sustained release of high doses, up to 300 mg, of the antiviral drug substantially improves patient compliance and reduce the likelihood of drug resistance.

Antiviral drugs such as protease inhibitors are solids with limited absorption, poor aqueous solubility and low bioavailability. The pharmaceutical composition comprising the liquid antiviral drug formulation described herein provides a sustained release oral dosage form to deliver a therapeutically acceptable level of the antiviral drug to a patient in need thereof. Additionally, the antiviral pharmaceutical composition provides improved bioavailability of the antiviral drug and is substantially independent of the variable environment of the gastrointestinal tract. The pharmaceutical composition comprises a liquid antiviral drug formulation in a sustained release dosage form, wherein the antiviral pharmaceutical composition is substantially free of *in-situ* aggregation effect of the antiviral drug, and provides substantially improved bioavailability of the antiviral drug. The pharmaceutical composition comprises an antiviral drug solubilized in a solvent, a hydrogel and optionally an osmagent and a lubricant.

The pharmaceutical composition comprises an antiviral drug in an amount of 5 wt% to 60 wt%; preferably 5 wt% to 55 wt%; and more preferably 10 wt% to 50 wt% of the total composition. Preferably, the antiviral drug formulation exists as a homogeneous blend, as a self-emulsifying formulation, as an emulsion or as micelles.

Examples of antiviral drugs include acyclovir, azidouridine, anasmycin, amantadine, bromovinyldeoxusidine, chlorovinyldeoxusidine, cytarbine, didanosine, deoxynojirmycin, dideoxycitidine, dideoxyinosine, dideoxynudeoside, desciclovir, deoxyacyclovir, edoxuidine, enviroxime, fiacitabine, foscarnet, fialuridine, fluorothymidine, fluxuridine, ganciclovir, hypericin, interferon, interleukin, isethionate, idoxuridine, nevirapine, pentamidine, ribavirin, rimantadine, stavirdine, sargramostin, suramin, trichosanthin, trifluorothymidine, tribromothymidine, trichlorothymidine, vidarabine, zidoviridine, zalcitabine and 3-azido-3-deoxythymidine.

Protease inhibitors inhibit human immunodeficiency virus (HIV) protease, the enzyme necessary for the maturation and the replication of the virus (Am J Health-Syst Pharm, Vol. 55, pp 233-254, (1998). Examples of protease inhibitors include, but are not limited to, saquinavir, adefovir, ritonavir, indinavir, nelfinavir, amprenavir, zidovudine and zalcitabin.

The pharmaceutical composition comprises an antiviral drug solubilized in a solvent. The solvents comprise surfactants, oil, liophilic components and mixtures thereof. The pharmaceutical composition comprises a solvent in an amount of 20 wt% to 95 wt%; preferably 20 wt% to 80 wt%; more preferably 30 wt% to 70 wt%; and even more preferably 40 wt% to 60 wt% of the total pharmaceutical composition.

Preferably, the solvent is a liquid surfactant, more preferably a non-ionic surfactant. Examples of surfactants include polysorbates, i.e., polyoxyethylene sorbitan fatty acid esters, such as polyoxyethylene 20 sorbitan monolaurate, wherein the number indicates the number of ethylene groups, polyoxyethylene 40 sorbitan monopalmitate, polyoxyethylene 60 sorbitan monostearate, and polyoxyethylene 80 sorbitan monooleate. The hydrophilic and lipophilic properties of polysorbates are useful for providing emulsions or micelles comprising antiviral drugs. The surfactants are known in Handbook of Pharmaceutical Excipients, edited by Wade and Weller, pp 375-378, (1994).

Examples of oils and lipophilic components for use as solvents in the pharmaceutical composition include superfine oils and triglycerides, such as almond oil, corn oil, cottonseed oil, menhaden oil, olive oil, orange roughy oil, peanut oil, safflower oil, sesame oil, shark liver oil, soybean oil, and the like; propylene glycol monoesters of fatty acids or glycerol fatty acid esters, such as capmuls, captexs, and the like; and distilled acetylated monoglycerides, such as myvacets, all of which are commercially available from Abitec, Croda or Eastman.

The pharmaceutical composition further comprises a hydrophilic polymer, such as, polyalkylene oxide, an alkali carboxyalklcellulose, hydroxyalkylcellulose, hydroxypropylalkylcellulose, and the like. The pharmaceutical composition comprises a hydrophilic polymer in an amount of 0 wt% to 40 wt%; preferably 0.1 wt% to 30 wt%; and more preferably 0.5 wt% to 20 wt% of the total pharmaceutical composition.

Preferred hydrophilic polymers comprise polyalkylene oxide of 25,000 to 750,000 number average molecular weight. Preferably, the pharmaceutical composition comprises 5 wt% to 40 wt% of the polyalkylene oxide selected from the group consisting of polyethylene oxide of 50,000 to 325,000 molecular weight, polyethylene oxide of 400,000 to 650,000 molecular weight, and a polypropylene oxide of 75,000 to 300,000 molecular weight; and an alkali carboxyalklcellulose of 25,000 to 750,000 number average molecular weight selected from the group consisting of poly(sodium carboxymethylcellulose), poly(potassium carboxymethylcellulose), poly(lithium carboxymethylcellose), and poly(sodium carboxyethylcellulose). The pharmaceutical composition may comprise additionally 0 wt% to 20 wt% of a hydroxypropylalkylcellulose of 9,000 to 1,250,000 number average molecular weight. The hydroxypropylalkylcellulose prevents particles from agglomerating and inhibits the formation of drug sediments. Examples of hydroxypropylalkylcellulose include hydroxypropylmethylcellulose, hydroxypropylethylcellulose, hydroxypropylbutylcellose, and hydroxypropylpentylcellulose. The pharmaceutical composition may comprise 0wt% to 10wt% of hydroxyalkylcellulose 10,000 to 1,150,000 number average molecular weight. Hydroxyalkylcellulose is used as a binder in the pharmaceutical composition. Examples of hydroxyalkylcellulose include, but are not limited to, hydroxymethylcellulose, hydroxyethylcellose, hydroxypropylcellulose, hydroxybutylcellulose, and hydroxypentylcellulose.

Additionally, the pharmaceutical composition comprises an osmagent. Osmagents are also referred to as osmotic solutes and osmotically effective agents, and are used interchangeably herein. The osmagents imbibe aqueous or biological fluids into the pharmaceutical dosage form thereby dispensing the antiviral drug dissolved or dispersed in the surfactant or its mixture with an oil. The pharmaceutical composition comprises an osmogent in an amount of about 0 wt% to 25 wt%; preferably 0.1 wt% to 20 wt%; and more preferably 0.5 wt% to 20 wt% of the total pharmaceutical composition.

Examples of osmagents include sodium chloride, potassium chloride, lithium chloride, magnesium sulfate, magnesium chloride, potassium sulfate, sodium sulfate, lithium sulfate, potassium acid phosphate, sodium phosphate, mannitol, urea, inositol, magnesium succinate, tartaric acid, raffinose, sucrose, glucose, lactose, urea, carbohydrates, water soluble inorganic salts, and water soluble organic salts. The osmotically effective solutes are known in U.S. Pat. No. 4,950,486.

Additionally, the pharmaceutical composition comprises a lubricant. The lubricant is used to enhance the manufacture of the pharmaceutical composition by lessening the incidence of ingredients adhering to manufacturing machinery. The pharmaceutical composition comprises a lubricant in an amount of about 0 wt% to 5 wt%; preferably 0.1 wt% to 5 wt%; and more preferably 0.5 wt% to 2 wt% of the total pharmaceutical composition. Examples of lubricants include magnesium stearate, calcium stearate, sodium stearate, stearic acid, glyceryl monostearate, glyceryl palmitostearate, zinc stearate, and talc. The total weight present, wt%, of all ingredients in the pharmaceutical composition is equal to 100 wt%.

The invention is directed to a sustained release oral dosage form comprising a pharmaceutical composition comprising the liquid antiviral drug formulation as described above, wherein the pharmaceutical composition is substantially free of *in-situ* aggregation effect of the antiviral drug and provides substantially improved bioavailability of the antiviral drug.

The sustained release oral dosage form comprises: (a) a wall defining a compartment, the wall comprising a semipermeable layer; (b) an expandable layer located within the compartment and in fluid communication with the semipermeable layer; (c).a capsule located within the compartment and in direct or indirect contacting relationship with the expandable layer, the capsule comprising the antiviral pharmaceutical composition as described above; and (d) an exit orifice formed or formable in the dosage form extending from the external surface of the capsule to the environment of use.

The pharmaceutical composition is manufactured into a dosage form by first adding the pharmaceutical composition to a capsule. The capsule can be classified according to conventional sizes including (000), (00), (0), (1), (2), (3), (4), and (5), wherein the diameter of the capsule is within the range of 10 mm to 3.0 mm. The inner capsule with the largest number has the smallest size. These capsules are disclosed in Microcapsule Processing and Technology, by Kondo and Van Vackenburg, page 2, (1979) published by Marcel Dekker, Inc. New York.

The capsule, in one manufacture, comprises two parts, a cap that slips over a body. The two parts are fitted together after the body is filled with the pharmaceutical composition. The assembly is done by slipping or telescoping the cap section over the body section, thereby completely surrounding and encapsulating the pharmaceutical composition. Capsules comprising a standard configuration are known in Pharmaceutical Sciences, by Remington, 14^{th} ed., pp 1671-1677, (1970), published by Mack Publishing Co.

Capsules can also be classified as soft capsules and as hard capsules. The soft capsule, as used by the present invention preferably in its final form, comprises one piece. Generally, the soft capsule is of sealed construction encapsulating the antiviral pharmaceutical composition therein. The soft capsule is made by various processes including the plate process, the rotary die process, the reciprocating die process, and the continuous process. The plate process uses a set of molds. A warm sheet of prepared capsule-wall forming material is laid over a lower mold and the pharmaceutical composition poured on it. The second sheet of wall-forming material is placed over the pharmaceutical composition followed by the top mold. The mold is placed under a press and a pressure applied, with or without heat to form a unit, soft capsule member. The capsules are washed with a solvent for removing excess antiviral pharmaceutical composition from the exterior of the capsule and the air-dried capsule is capsuled with an expandable layer, also referred to as a hydroactivated layer, comprised of a hydroactivated composition.

A hard capsule is composed of two parts, a cap and a body, which are fitted together after the larger body is filled with a preselected appropriate antiviral pharmaceutical composition. This is done by slipping or telescoping the cap selection over the body section, thus completely surrounding and encapsulating the pharmaceutical composition. A hard capsule is made by dipping stainless steel molds into a bath containing solution of a capsule wall-forming material to coat the mold with the material. Then, the molds are withdrawn, cooled, and dried in a current of air. The capsule is stripped from the mold and trimmed to yield the capsule with an internal lumen. The engaging caps that telescopically caps the antiviral pharmaceutical composition receiving body is made in a similar manner. Then, the closed and filled capsule is capsulated with an expandable layer comprised of a hydroactivated composition an outer semipermeable membrane.

The hard capsule can also be made with each part having matched locked rings near the opened end that permits joining and locking together the overlapping cap and body after filling with the pharmaceutical composition.

A pan of matched locking rings are formed into the cap portion and the body portion and these rings provide the locking means for security holding together the capsule. The capsule can be manually filled or machine filled with the pharmaceutical composition. In the final manufacture, the hard capsule is capsulated with a semipermeable layer comprised of a semipermeable composition permeable to the passage of aqueous and biological fluids, and impermeable to the passage of ingredients contained in the capsule.

The rotary die process for providing a capsule comprises two continuous films of capsule wall-forming materials that are brought into convergence between a pan of revolving dies and an injector wedge. The process fills and seals the capsule in dual and coincident operations. In this process, the sheets of capsule forming compositions are fed over guide rolls, and then down between the wedge injector and the die rolls. The pharmaceutical composition to be capsulated flows by gravity into a positive displacement pump. The pump meters the pharmaceutical composition through the wedge injector and into the sheets between the die rolls. The bottom of the wedge contains small orifices lined-up with the die pickets of the die rolls. The capsule is about half-sealed when the pressure of pumped pharmaceutical composition forces the sheets into the die pockets. Wherein the soft capsules are simultaneously filled, shaped, hermetically sealed and cut from the sheets of wall-forming compositions. The sealing of the soft capsule is achieved by mechanical pressure on the die rolls and by heating the sheets of wall-forming compositions by the wedge. After manufacture, the antiviral pharmaceutical composition-filled capsules are dried in the presence of forced air.

The reciprocating die process produces soft capsules by leading two films of capsule wall-forming compositions between a set of vertical dies. The dies as they close, open, and close, perform as a continuous vertical place forming row after row of pockets across the film. The pockets move through the dies, they are sealed, shaped and cut from the moving film as capsules filled with pharmaceutical composition. An expandable layer and a semipermeable layer are coated thereon to yield the osmotic dosage system. The continuous process is a manufacturing system that also uses rotary dies with the added feature that the process can successfully fill an antiviral pharmaceutical composition into a soft capsule to encapsulating liquids therein.

The capsules, used for the purpose of this invention comprise a gelatin wall. The gelatin comprises a viscosity of 15 to 30 millipoises and a bloom strength up to 150 grams; gelatin having a bloom value of 160 to 250; a composition comprising gelatin, glycerine, water and colorant titanium dioxide; a composition comprising gelatin, erythrosin, iron oxide and titanium dioxide; a composition comprising gelatin, glycerine, sorbitol, potassium sorbate, and titanium dioxide; and a composition comprising gelatin, acacia, glycerine and water. Materials useful for forming capsules are known in U.S. at. Nos. 4,627,850 and 4,663,148.

The capsule, in one manufacture is filled with the antiviral pharmaceutical composition and the capsule cap slid over the capsule body, then coated on its exterior surface with a semipermeable layer. The semipermeable layer form a membrane on the exterior surface of the capsule, and maintains its physical and chemical integrity during operation of the dosage form and is essentially-free of interaction with the gelatin capsule.

The semipermeable membrane comprises a semipermeable polymer, including a semipermeable homopolymer and a semipermeable copolymer. In preferred embodiment, the semipermeable polymers comprise cellulose esters, cellulose ethers, and cellulose ester-ethers. The cellulosic polymers have a degree of substitution. D.S. on their anhydroglucose unit from greater than 0 up to 3 inclusive. By degree of substitution is meant the average number of hydroxyl groups originally present on the anhydroglucose unit that are replaced by a substituting group, or converted into another group. The anhydroglucose unit can be partially or completely substituted with groups such as acyl, alkanoyl, alkenoyl, aroyl, alkyl, alkoxy, halogen, carboaalkyl, alkylcarbamate, alkylcarbonate, alkylsulfonate, alkylsulfamate, semipermeable polymer forming groups, and the like.

Additional examples of semipermeable polymers include cellulose acylate, cellulose diacylate, cellulose triacylate, cellulose acetate, cellulose diacetate, cellulose triacetate, mono, di- and tri-cellulose alkanylates, mono-, di-, and tri-alkenylates, mono-, di-, and tri-aroylates, and the like. Exemplary polymers include cellulose acetate have a D.S. of 1.8 to 2.3 and an acetyl content of 32 to 39.9%; cellulose diacetate having a D.S. of 1 to 2 and an acetyl content of 21 to 35%, cellulose triacetate having a D.S. of 2 to 3 and an acetyl content of 34 to 44.8%, and the like. More specific cellulosic polymers include cellulose propionate having a D.S. of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a D.S. of 1.8, an acetyl content of 13 to 15%, and a butyryl content of 34 to 39%; cellulose acetate butyrate having an aacetyl content of 2 to 29%, a butyryl content of 17 to 53%, and a hydroxyl content of 0.5 to 4.7%; cellulose triacylates having a D.S. of 2.6 to 3 such as cellulose trivalerate, cellulose tripalmate, cellulose tristearate cellulose trioctanoate, and cellulose tripropionate; cellulose diesters having a D.S. of 2.2 to 2.6 such as cellulose disuccinate, cellulose dipalmitate, cellulose diotanoate, cellulose dicarpylate, and the like; mixed cellulose esters such as cellulose acetate valerate, cellulose acetate succinate, cellulose propionate succinate, cellulose acetate octanoate, cellulose valerate palmitate, cellulose acetate heptonate, and the like. Semipermeable polymers are known in U.S. at. No. 4,077,407 and they can be synthesized by procedures described in Encyclopedia of Polymer Science and Technology, Vol. 3, pages 325 to 354, 1964, published by Interscience Publishers, Inc. New York.

Additional semipermeable polymeric composition for forming a membrane that surrounds the capsule comprise cellulose acetaldehyde dimethyl acetate; cellulose acetate ethylcarbamate; cellulose acetate methylcarbamate; cellulose dimethylaminoacetate; semipermeable polyamide; semipermeable polyurethanes; semipermeable sulfonated polystyrenes; cross-linked selectively semipermeable polymers formed by the coprecipitation of a polyanion and a polycation as disclosed in U.S. Pat. Nos. 3,173,876; 3,276,586; 3,541,005; 3,541,006; and 3,546,142; semipermeable polymers as disclosed by Loeb et al in U.S. Pat. No. 3,133,132; semipermeable poly (sodium styrenesulfonate); semipermeable poly (vinylbenzyltremethylammonium chloride); semipermeable polymers, exhibiting a fluid permeability of 10⁻⁵ to 10⁻² (cc. mil/cm hr.atm) expressed as per atmosphere of hydrostatic or osmotic pressure differences across a semipermeable wall. The polymers are known to the art in U.S. Pat. Nos. 3,845,770; 3,916,899, and 4,160,020 and in Handbook of Common Polymers, by Scott, J.R. and Roff, W.J., 1971, published by CRC Press, Cleveland, Ohio.

The dosage form comprises an expandable layer comprised of a hydroactivated composition. In certain embodiments of the dosage form, the expandable layer is located within the capsule and is remote from the exit orifice. The pharmaceutical composition is added first into the body of the capsule. Then, the expandable layer is positioned in contact with the pharmaceutical composition followed by closing the capsule with the cap. The expandable layer is a push-displacement osmotic composition, and it operates to push and displaces the pharmaceutical composition through an exit passageway from the dosage form. In alternative embodiments of the dosage form, the expandable layer is located within the compartment between the capsule and the semipermeable layer. The expandable layer comprises hydrophilic polymers also known as hydrogels or osmopolymers. The osmopolymers exhibit fluid imbibition properties. The osmopolymers are swellable, hydrophilic polymers, which osmopolymers interact with water and biological aqueous fluids and swell or expand. The osmopolymers exhibit the ability to swell in water and biological fluids and retain a significant portion of the imbibed fluid within the polymer structure. The osmopolymers swell or expand to a very high degree, usually exhibiting a 2 to 60 fold volume increase. The osmopolymers can be noncross-linked or cross-linked. The swellable, hydrophilic polymers may be lightly cross-linked, such cross-links being formed by covalent or ionic bonds or residue crystalline regions after swelling. The osmopolymers can be plant, animal or synthetic origin. The osmopolymers are hydrophilic polymers. Hydrophilic polymers suitable for the present purpose include poly (hydroxyalkyl-mydroxyalkyl methacrylate) having a molecular weight of from 30,000 to 5,00,000; polyalkylene oxide of 1,500,000 to 10,000,000 number average molecular weight including polyethylene oxide of 5,000,000 molecular weight, and polyethylene oxide of 7,800,000 molecular weight; alkali carboxyalkylcellulose of 450,000 to 7,500,000 number average molecular weight represented by a member selected from the group consisting of sodium carboxymethylcellulose, potassium carboxymethylcellulose and lithium carboxymethylcellulose. The hydrogels comprise anionic and cationic hydrogels polyelectrolyte complexes; a mixture of methylcellulose, agar and sodium carboxymethylcellulose; a mixture of hydroxypropylmethylcellulose and sodium carboxymethylcellulose; a mixture of hydropropylethylcellulose and sodium carboxymethylcellulose; polyoxyethylenepolyoxypropylene gel; polyoxybutylene-polyethylene block copolymer gel; carob gum; polyacrylic gel; polyester gel; polyuria gel; polyether gel; polyamide gel; polycellulosic gel; polygum gel; initially dry hydrogels that imbibe and absorb water which penetrates the hydrogel and lowers its glass temperature; and the like.

Representative of other osmopolymers comprise polymers that form hydrogels such as Carbopol® acidic carboxypolymer, a polymer of acrylic and cross-linked with a polyallyl sucrose, also known as carboxypolymethylene and carboxyvinyl polymer having a molecular weight of 250,000 to 4,000,000. Cyunamer®polyacrylamides; cross-linked water swellable indenemaleic anhydride polymers; Good-rite® polyacrylic acid having a molecular weight of 80,000 to 200,000 polyethylene oxide polymers of 100,000 to 7,800,000 molecular weight blended with gums such as guar gum; starch graft copolymers; Aqua-Keps® acrylate polymer polysaccharide composed of condensed glucose units such as diester cross-linked polyglucaride; and the like. Representative polymers that form hydrogels are known to the prior art in U.S. Pat. No. 3,865,108 issued to Hartop; U.S. Pat. No. 4,002,173 issued to Manning; U.S. Pat. No. 4,207,893 issued to Michaels, and in Handbook of Common Polymers, by Scott and Roff, published by the Chemical Rubber Co., Cleveland, Ohio.

Additional examples of hydrophilic polymers comprise hydrophilic cellulose, such as, hydroxypropylmethylcellulose, hydroxypropylethylcellulose, hydroxypropylbutylcellulose and hydroxypropylpentylcellulose, the amount of the hydroxypropylalkylcellulose present in the push-displacement composition is 0 wt% to 25 wt%. The hydroxypropylalkylcellulose imparts cohesive qualities to a pharmaceutical composition or to the expandable layer. The expandable layer comprises a hydroxyalkylcellulose such as hydroxyalkylcellulose, hydroxymethylcellulose, hydroxyethylcellulose, hydroxypropylcellulose, hydroxybutylcellulose, and hydroxyhexylcellulose. The amount of hydroxyalkylcellulene present in the expandable layer is 0 wt% to 15 wt%. The hydroxyalkylcellulose serves as a binder and as a stabilizing agent. The hydroxypropylcellulose are commercially available as Klucel EF of 80,000 molecular weight; Klucel® LF of 95,000 molecular weight; Klucel JF of 140,000 molecular weight; Klucel GF of 370,000 molecular weight; Klucel MF of 850,000 molecular weight; and Klucel HF of 1,150,000 molecular weight. The hydroxypropylcellulose are disclosed in Handbook of Pharmaceutical Excipients, 2^{nd} ed., Edited by Wade and Weller, pp 223-228, (1994).

The expandable layer comprises a hydrophilic polymer in an amount of 0 wt% to 95 wt%; preferably 10 wt% to 70 wt%; more preferably 25 wt% to 70 wt%; and even more preferably 50 wt% to 70 wt% of the total composition of the expandable layer.

The expandable layer further comprises an osmotically effective compound comprising inorganic and organic compounds that exhibit an osmotic pressure gradient across the semipermeable membrane, against an external fluid. The osmotically effective compounds, as with the osmopolymers, imbibe fluid into the osmotic system, thereby making available displacement-push to push the pharmaceutical composition from the osmotic dosage form. The osmotically effective solutes are known also as osmagents and include magnesium sulfate, magnesium chloride, potassium sulfate, sodium sulfate, lithium sulfate, potassium acid phosphate, mannitol, urea, inositol, magnesium succinate, tartaric acid, carbohydrates, raffinose, sucrose, glucose, lactose, and sorbitol. Osmotically effective solutes are taught in U.S. Pat. No. 4,783,337. The expandable layer comprise an osmotically effective solute in an amount of about 0 wt% to about 60 wt%; preferably about 5 wt% to about 55 wt%; more preferably about 10 wt% to about 40 wt%; and even more preferably about 20 wt% to about 30 wt% of the total composition of the expandable layer.

The expandable layer optionally comprises a lubricant, which prevents or reduces adhesion of the composition to the surfaces of dies and punches. The lubricant comprises calcium stearate, zinc stearate, magnesium stearate, magnesium oleate, calcium palmitate, sodium suberate, potassium laurate, stearic acid, salts of fatty acids, salts of alicylic acids, salts of aromatic acids, oleic acid, palmitic acid, and a mixture of a salt of a fatty, alicyclic, or aromatic acid. The amount of lubricant in a hydro-activated, push-displacement composition is 0.01 wt% to 4.5 wt%. The weight of all ingredients in this composition is 100 wt%.

The pharmaceutical composition and the expandable layer can comprise 0 wt% to 3 wt% of a nontoxic colorant. The colorant makes the composition and the composition more esthetic in appearance, and the colorant serves to identify the parts of the dosage form during manufacture and therapy. The pharmaceutical composition and the expandable layer comprise a different colorant. The colorants are represented by FD&C Red No. 3; FD&C Red No. 40; FD&C Yellow No. 5; FD&C Yellow No. 6; FD&C Blue No. 1; FD&C Blue No. 2; FD&C Green No. 3; iron oxide; and titanium dioxide.

The expression "exit passageway" as used herein comprises means and methods suitable for releasing the beneficial agent from the osmotic system. The expression includes aperture, orifice, hole, bore, pore, porous element, porous overlay, porous insert, hollow fiber, capillary tube, microporous insert, microporous overlay, and the like. The passageway can be formed by mechanical drilling, laser drilling, eroding an erodible element, extracting, dissolving, bursting, or leaching a passageway former from the wall. The passageway can be a pore formed by leaching sorbitol, lactose or the like from a wall or layer as disclosed in U.S. Pat. No. 4,200,098. This patent discloses pores of controlled-size porosity formed by dissolving, extracting, or leaching a material from a wall, such as sorbitol from cellulose acetate. The pore-passageways extend from the inside to the outside of a wall or layer for effective release of beneficial agent including a drug to the exterior of the osmotic system. U.S. Pat. No. 4,285,987 discloses an osmotic system comprising a first osmotic system comprising a cellulose acetate wall comprising leachable sorbitol for forming a pore for releasing an osmotically active beneficial agent from an osmotic core. This patent, discloses an osmotic system that exhibits drug released through a pore-passageway and drug released through a laser-drilled passageway within the total structure of the same osmotic system. Passageways are known in U.S. Pat. No. 4,783,337.

The dosage form is provided by coating a capsule with a semipermeable composition. The semipermeable composition can be applied to the exterior surface of the capsule by molding, spraying, dipping, or the like the capsule into a semipermeable coat-forming composition. Another technique that can be used for applying the semipermeable composition is the air suspension technique. This technique consists in suspending and tumbling the composition in a current of air until the semipermeable composition surrounds and coats the capsule. The air suspension technique is described in U.S. Pat. No. 2,799,241; J. Am. Pharm. Assoc., Vol. 48, pp 451-459, 1979, and ibid, Vol. 49, pp 82-84, 1960. Other standard manufacturing procedures are described in Modem Plastic Encyclopedia, Vol. 46, pp 62-70, 1969, and in Pharmaceutical Sciences, by Remington 14^{th} Ed., pp 1626-1678, 1970, published by Mack Publishing Co., Easton, PA.

Representative of inorganic solvents and organic solvents for coating the capsule are solvents that do not adversely harm the materials, the capsule and the semipermeable coated capsule. The solvents broadly include members selected from the group consisting of aqueous solvents, alcohols, ketones, esters, ethers, aliphatic hydrocarbons, halogenated solvents, cycloaliphatic, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride acetone and water, acetone and methanol, acetone and ethyl alcohol, methylene dichloride and methanol, and ethylene dichloride and methanol.

In alternative embodiments, the dosage form further comprises a barrier layer. In certain embodiments of the dosage form, the expandable layer is located within the capsule and is remote from the exit orifice, and the barrier layer is located within the capsule between the antiviral pharmaceutical composition and the expandable layer. In alternative embodiments of the dosage form, the expandable layer is located within the compartment between the capsule and the semipermeable layer, and the barrier layer is located within the compartment between the capsule and the expandable layer.

Suitable materials for forming the barrier layer may include, for example, polyethylene, polystyrene, ethylene-vinyl acetate copolymers, polycaprolactone and Hytrel® polyester elastomers (Du Pont), cellulose acetate, cellulose acetate pseudolatex (such as described in U.S. Patent 5,024,842), cellulose acetate propionate, cellulose acetate butyrate, ethyl cellulose, ethyl cellulose pseudolatex (such as Surelease® as supplied by Colorcon, West Point, PA or Aquacoat™ as supplied by FMC Corporation, Philadelphia, PA), nitrocellulose, polylactic acid, polyglycolic acid, polylactide glycolide copolymers, collagen, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters (such as Eudragit® supplied by RöhmPharma, Darmstaat, Germany), polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyalkoxysilanes, polydimethyl siloxane, polyethylene glycol-silicone elastomers, electromagnetic irradiation crosslinked acrylics, silicones, or polyesters, thermally crosslinked acrylics, silicones, or polyesters, butadiene-styrene rubber, and blends of the above.

Preferred materials include cellulose acetate, copolymers of acrylic acid and methacrylic acid esters, copolymers of methylmethacrylate, and ethylacrylate, and latex of acrylate esters. Preferred copolymers include poly (butyl methacrylate), (2-dimethylaminoethyl)methacrylate, methyl methacrylate) 1:2:1, 150,000, sold under the trademark EUDRAGIT E; poly (ethyl acrylate, methyl methacrylate) 2:1, 800,000, sold under the trademark EUDRAGIT NE 30 D; poly (methacrylic acid, methyl methacrylate) 1:1, 135,000, sold under the trademark EUDRAGIT L; poly (methacrylic acid, ethyl acrylate) 1:1, 250,000, sold under the trademark EUDRAGIT L; poly (methacrylic acid, methyl methacrylate) 1:2, 135,000, sold under the trademark EUDRAGIT S; poly (ethyl acrylate, methyl methacrylate, trimethylammonioethyl methacrylate chloride) 1:2:0.2, 150,000, sold under the trademark EUDRAGIT RL; poly (ethyl acrylate, methyl methacrylate, trimethylammonioethyl methacrylate chloride) 1:2:0.1. 150,000, sold as EUDRAGIT RS. In each case, the ratio x:y:z indicates the molar proportions of the monomer units and the last number is the number average molecular weight of the polymer. Especially preferred are cellulose acetate containing plasticizers such as acetyl tributyl citrate and ethylacrylate methylmethylacrylate copolymers such as Eudragit NE.

The foregoing materials for use as the barrier layer may be formulated with plasticizers to make the barrier layer suitably deformable such that the force exerted by the expandable layer 20 will collapse the compartment formed by the barrier layer 18 and gelatin capsule 12 to dispense the liquid, active agent formulation. Examples of typical plasticizers are as follows: polyhydric alcohols, triacetin, polyethylene glycol, glycerol, propylene glycol, acetate esters, glycerol triacetate, triethyl citrate, acetyl triethyl citrate, glycerides, acetylated monoglycerides, oils, mineral oil, castor oil and the like. The plasticizers may be blended into the material in amounts of 10-50 weight percent based on the weight of the material. The barrier layer and its composition is described in the international publication WO 00/35419, which is incorporated herein by reference.

In preferred embodiments, the expandable layer is located within the capsule and is remote from the exit orifice.

Preferably, the capsule is a gelatin capsule.

### Methods Of Using The Invention

There is also described a method for administering a beneficial protease inhibitor at a controlled rate to the gastrointestinal tract of a human. The method not part of the invention comprises: (A) admitting orally into the gastrointestinal tract a dosage form comprising: (1) a capsule comprising a single body or a capsule comprising a body and a matching cap telescopically joined to define a capsule comprising a lumen; (2) a pharmaceutical composition in the capsule comprising a protease inhibitor that is self-emulsified in the presence of a liquid nonionic surfactant; (3) a membrane that surrounds the capsule comprising a semipermeable polymer permeable to the passage of an aqueous or biological fluid, and impermeable to a protease inhibitor; and, (4) an exit passageway through the semipermeable membrane for delivering the protease inhibitor from the dosage form; and wherein the method comprises: (B) admitting orally into the gastrointestinal tract a dosage form comprising: (1) a capsule comprising a single body, or a capsule comprising a body and a matching cap telescopically engaged to form a capsule comprising an internal lumen; (2) a pharmaceutical composition in the capsule comprising a protease inhibitor that is solubilized or dispersed in a liquid nonionic surfactant or its mixture with a lipophilic component; (3) an osmotic layer in the capsule comprising a member selected from the group consisting of an osmopolymer and an osmagent; (4) a membrane that surrounds the capsule, the membrane comprising a semipermeable composition permeable to the passage of an aqueous or a biological fluid present in an environment of use; (5) an orifice through the exterior membrane that communicates with the capsule; (C) imbibing fluid, in both dosage forms, through the semipermeable membrane into the capsule at a rate determined by the permeability of the semipermeable composition membrane, and the osmotic pressure gradient across the membrane thereby causing the pharmaceutical composition to be osmotically and hydrodynamically pumped from the dosage form; and (D) delivering the protease inhibitor through the exit at a controlled-sustained rate to the patient over a prolonged period of time.

Also described is a method not part of the invention of treating a condition in a subject responsive to antiviral medication, the method comprising orally administering to the subject a sustained release dosage form comprising an antiviral pharmaceutical composition wherein the composition is substantially free of *in-situ* aggregation effect of the antiviral drug and provides substantially improved bioavailability of the antiviral drug. Preferably, the dosage form administers a therapeutically effective dose of the antiviral drug over a period of at least 4 hours after oral administration with no more than 30% by weight of the liquid composition being released within the first 1 hour after oral administration. In alternative embodiments, the dosage form administers a therapeutically effective dose of the antiviral drug over a period of at least 12 hours after oral administration with no more than 30% by weight of the liquid composition being released within the first 4 hours after oral administration. In additional embodiments, the dosage form administers a therapeutically effective dose of the antiviral drug over a period of 24 hours after oral administration with no more than 30% by weight of the liquid composition being released within the first 12 hours after oral administration. The dosage form produces an average steady-state plasma concentration of the antiviral drug greater than the therapeutically effective concentration of the antiviral drug over a period of between about 4 hours to about 24 hours.

### II. Experimental

Below are examples of specific embodiments for carrying out the present invention. The examples are offered for illustrative purposes only, and are not intended to limit the scope of the present invention in any way, as these examples and other equivalents thereof will become more apparent to those versed in the art in the light of the present disclosure, the drawing figures and the accompanying claims.

Efforts have been made to ensure accuracy with respect to numbers used (e.g., amounts, temperatures, etc.), but some experimental error and deviation should, of course, be allowed for.

### Example 1

A dosage for the controlled delivery of a liquid pharmaceutical composition is manufactured as follows: first, an osmotic push-displacement composition is prepared using a fluid bed granulation. The osmagent sodium chloride is sized and screened in a mill using a 21-mesh screen. Then, the following dry ingredients are added into the granulation bowl: 58.75% sodium carboxymethylcellulose, 30% sodium chloride, 5.0% hydroxypropylmethylcellulose possessing an 11,200 number average molecular weight, and 1.0% colorant ferric oxide. All the ingredients are blended in the granulator bowl. Next, in a separate container, a granulating solution is prepared by dissolving 5.0% hydroxypropylcellulose possessing an 80,000 molecular weight in purified water. Then, the granulating solution is sprayed onto the fluidized powders until all the granulating solutions is applied and the powders are granular. Then, 0.25% magnesium stearate lubricant is added to the granules and blended to provide a homogenous composition. The granules are compressed into a tablet with a standard tableting press. Two hundred and fifty mg (milligrams) of the granules are added to a 7.14 mm punch, tamped and then compressed under a force of 1 metric ton into a core.

Next, a pharmaceutical composition is prepared by homogeneously mixing 50 wt% nelfinavir and 50 wt% polyoxyethylene 20 sorbitan monooleate in a homogenizer.

Then, a gelatin capsule, size 0, is separated into as body and cap sections. Next, 600 mg of the pharmaceutical composition comprising the antiviral nelfinavir is added to the capsule body. Then, the osmotic tablet, prepared above is placed at the top of the pharmaceutical composition. The filled capsule is closed with the capsule cap.

Next, the assembled closed capsule is coated with a membrane on its exterior surface. The membrane possesses rate controlling properties and it assists in providing the controlled-sustained release dosage form. The membrane forming composition comprises 85% cellulose acetate having a 39.8% acetyl content, and 15% polyoxypropylene glycol consisting of 14,600 molecular weight and 280 moles of ethylene oxide. The membrane forming ingredients is dissolved in acetone to provide a 4% solid solution. The solution is sprayed around the closed capsule in a standard coater to provide a membrane weighing 42 mg. The membrane coated capsules are dried at 40°C and ambient relative humidity over night to evaporate residual solvent. Next, an exit passageway is drilled through the exterior membrane to provide a 15 mil (0.38 mm) passageway. The passageway connects the pharmaceutical composition with the environment of use.

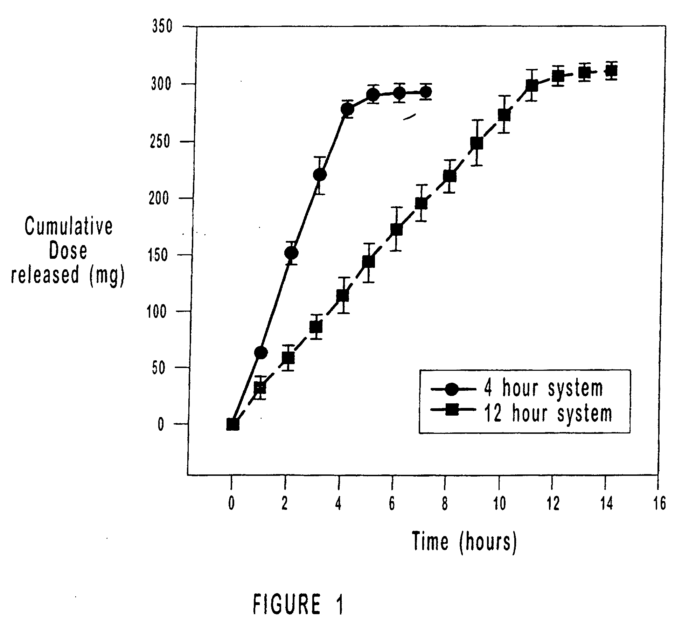

The dosage form controlled-sustained release drug delivery profile is measured in artificial gastric fluid without enzyme. Accompanying Figure 1, illustrates the cumulative amount of nelfinavir release from the liquid dosage form provided by this example and further identified by squares connected through a line for a 12 hour dosage form (system).

### Example 2

The procedure of Example 1 is followed in this example, with all conditions as previously described, except in this example the membrane weighted 43 mg and comprised 70% cellulose acetate comprising an acetyl content of 39.8% and 30% polyoxypropylene glycol. The pharmaceutical composition weighted 600 mg and comprises 300 mg of nelfinavir. Accompanying Figure 1 shows 90% of the protease inhibitor is delivered in 4 hours at a constant rate, as seen with circles connected by a continuous line.

### Example 3

The procedure of Example 1 is followed in this example, with the manufacturing steps comprising fluid bed granulation to provide a push-displacement composition, compressing the freshly prepared composition into an osmotic layer that is sized, shaped and adapted for placing in a capsule, separately blending a protease inhibitor selected from the group consisting of saquinavir, adefovir, ritonavir, indinavir, nelfinavir, amprenavir, zidovudine and zalcitabin, with a non-ionic surfactant to provide a pharmaceutical composition, wherein in situ, the composition intakes an aqueous or a biological fluid causing the protease inhibitor to be solubilized in the nonionic surfactant, assemblying the dosage form, by first adding the protease inhibitor nonionic surfactant composition, then placing the osmotic layer in the body of a capsule, capping the capsule, coating the closed capsule with a semipermeable membrane, and drying the coated capsule to remove residual solvent to provide the dosage form and then manufacturing an exit orifice in the dosage form.

### Example 4

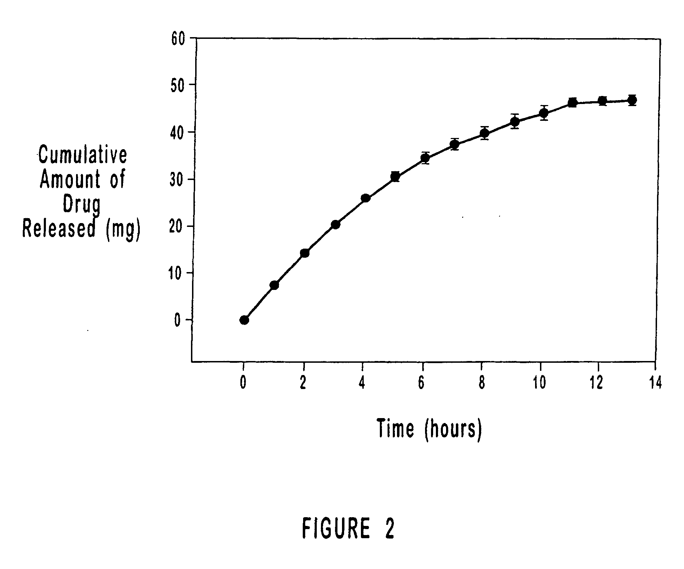

A dosage form for administering a protease inhibitor for treating a viral infection in a patient in need of a protease inhibitor therapy is prepared as follows: first, a pharmaceutical composition comprising 50 mg ritonavir, which is retrieved from a commercial product Norvir® capsule is filled into the body of a solution 0 size capsule. Then, an osmotic tablet, as described in Example 1, is placed on top of the pharmaceutical composition: next, the capsule closed is coated with a semipermeable membrane, and an exit passageway formed in the semipermeable membrane. The membrane comprising 80% cellulose acetate containing 39% acetyl content and 20% Poloxamer® 338, a commercially available emulsifier, weighs 152 mg. The protease inhibitor is delivered from the dosage form at a controlled rate over 12 hrs (Fig. 2).

### Example 5

A dosage form is manufactured according to claim 4, wherein 300 mg of a protease inhibitor selected from saquinavir, adefovir, ritonavir, indinavir, nelfinavir, amprenavir, zidovudine and zalcitabin is dispersed in a liquid carrier composed of a non-ionic surfactant and a mono-,di-triglyceride. Figure 2 illustrates a release profile for a delivery system provided by this invention.

### Example 6

A dosage form is manufactured according to the procedure of Example 4, wherein in this example the liquid nonionic surfactant is a member selected from the group consisting of sorbitan monolaurate polyoxyethylene also known as Tween® 20; and sorbitan mono-oleate polyoxyethylene also known as Tween® 80. Cremophor EL a polyoxyethylene 35 castor oil, Cremophor RH, a glycerol polyethylenglycol oxystearate, or the mixture of the Cremophor with Labrasol, a saturated polyglycolyzed glyceride commercially available from Gattefossfe Inc., or the mixture of the Cremophors with Myvacet 945, an acetylated monoglyceride, commercially available from Eastman Chemical Co.

### Example 7

A dosage form is manufactured according to the procedure of Example 4, with all conditions as set forth, except in this example the hydrogel is sodium carboxymethylcellulose of 40,000 number average molecular weight.

Accordingly, an antiviral pharmaceutical composition, a dosage form comprising the antiviral composition and a method of treatment are disclosed.

## Claims (Claims for the following Contracting State(s): AT, BE, CH, DE, DK, ES, FR, GB, GR, IE, IT, LI, LU, MC, NL, PT, SE, FI, CY)

1. A sustained release oral dosage form comprising:
(a) a wall defining a compartment, the wall comprising a semipermeable layer;
(b) an expandable layer located within the compartment and in fluid communication with the semipermeable layer;
(c) a capsule located within the compartment and in direct or indirect contacting relationship with the expandable layer, the capsule comprising a liquid antiviral drug composition comprising an antiviral drug solubilized in a solvent, wherein said antiviral drug is present in an amount of 5 wt% to 60 wt% and the solvent is present in an amount of 20 wt% to 95 wt% of the total antiviral drug composition, which composition is substantially free of *in-situ* aggregation effect of the antiviral drug and provides substantially improved bioavailability of said antiviral drug; and
(d) an exit. orifice formed or formable in the dosage form extending from the external surface of the capsule to the environment of use;
with the exclusion of a sustained release oral dosage as defined in this claim, but wherein:
(A) said wall is formed from a wall-forming composition comprising 85 wt% cellulose acetate comprising a 39.8 wt% acetyl content and 15 wt% polyethylene glycol 3350, said wall composition having been dissolved in acetone/methanol (80/20 wt/wt) cosolvent to make a 4% solid solution and having been sprayed onto said capsule in an air suspension coater, the resulting semipermeable-wall-coated capsules having been dried in an oven at 50°C and 50° relative humidity for 1 day to remove all the solvents; and
(B) said expandable layer consists of 250 mg of granules which have been compressed into a tablet-shaped layer in a 9/32 inch punch, tamped and then compressed under a force of 1 metric ton, said granules having been prepared by (i) spraying in a granulator the fluidized powders of 30 wt% sodium chloride screened through a 20 mesh screen, 58.75 wt% polyethylene oxide of 2,000,000 molecular weight, 5 wt% hydroxypropylmethylcellulose of 9,200 molecular weight and 1 wt% red ferric oxide, with 5 wt% hydroxypropylcellulose dissolved in purified water, and (ii) blending the granules obtained in (i) with 0.25 wt% magnesium stearate; and
(C) said capsule comprises a liquid antiviral drug composition consisting of an homogenous blend of 50 wt% microfluidized acyclovir, 12.5 wt% polyoxyethylene 20 stearate, 25 wt% polyoxyl 35 castor oil and 12.5 wt% polyoxyl 40 stearate, the capsule body having been charged first with said liquid antiviral drug composition, then with a piston formed of high density polyethylene, then with the expandable layer, the filled capsule body having then been closed with the gelatin cap; and
(D) said exit orifice has been drilled in said wall.

2. A dosage form according to claim 1 wherein the expandable layer is located within the capsule and is remote from the exit orifice.

3. A dosage form according to claim 2 further comprising a barrier layer located within the capsule between the antiviral drug composition and the expandable layer.

4. A dosage form according to claim 1 wherein the expandable layer is located within the compartment between the capsule and the semipermeable layer.

5. A dosage form according to claim 4 further comprising a barrier layer located within the compartment between the capsule and the expandable layer.

6. A dosage form according to any preceding claim wherein said semipermeable layer comprises a semipermeable polymer; and the expandable layer comprises a hydrophilic polymer.

7. A dosage form according to claim 6 wherein the expandable layer further comprises a lubricant and/or an osmotically effective compound.

8. A dosage form according to claim 6 or 7 wherein the hydrophilic polymer is present in an amount of up to 95 wt%; the osmotically effective agent is present in an amount of 0 wt% to 60 wt%; and the lubricant is present in an amount of 0 wt% to 5 wt% of the total composition of the expandable layer.

9. A dosage form according to any preceding claim wherein said solvent comprises a surfactant, an oil or mixtures thereof.

10. A dosage form according to claim 9 wherein said surfactant is a non-ionic surfactant.

11. A dosage form according to any preceding claim wherein said liquid antiviral drug composition comprises a hydrogel and/or an osmagent.

12. A dosage form according to any preceding claim wherein the antiviral drug is selected from the group consisting of acyclovir, azidouridine, anasmycin, amantadine, bromovinyldeoxusidine, chlorovinyldeoxusidine, cytarbine, didanosine, deoxynojirmycin, dideoxycitidine, dideoxyinosine, dideoxynudeoside, desciclovir, deoxyacyclovir, edoxuidine, enviroxime, fiacitabine, foscarnet, fialuridine, fluorothymidine, fluxuridine, ganciclovir, hypericin, interferon, interlenkin, isethionate, idoxuridine, nevirapine, pentamidine, ribavirin, rimantadine, stavirdine, sargramostin, suramin, trichosanthin, trifluorothymidine, tribromothymidine, trichlorothymidine, vidarabine, zidoviridine, zalcitabine, 3-azido-3-deoxythymidine, saquinavir, adefovir, ritonavir, indinavir, nelfinavir, amprenavir, zidovudine and zalcitabin.

13. A dosage form according to any preceding claim wherein said antiviral drug is a protease inhibitor.

14. A dosage form according to any preceding claim wherein the capsule is a gelatin capsule.

15. A dosage form according to claim 14 which can produce an average steady-state plasma concentration of the antiviral drug greater than the therapeutically effective concentration of the antiviral drug over a period of about 4 hours to about 24 hours.

16. A dosage form according to any preceding claim for use in treating a condition in a subject responsive to the antiviral drug, wherein said condition is acquired immune deficiency syndrome (AIDS) associated with human immunodeficiency virus (HIV) infection in the subject.

17. A dosage form according to any preceding claim which can administer a therapeutically effective dose of said antiviral drug over a period of at least 4 hours after administration with no more than 30% by weight of said drug composition being released within the first 1 hour after oral administration.

18. A dosage form according to any one of claims 1 to 16 which can administer a therapeutically effective dose of said antiviral drug over a period of at least 12 hours after administration with no more than 30% by weight of said drug composition being -released within the first 4 hours after oral administration.

19. A dosage form according to any one of claims 1 to 16 which can administer a therapeutically effective dose of said antiviral drug over a period of 24 hours after administration with no more than 30% by weight of said drug composition being released within the first 12 hours after oral administration.

## Claims (Claims for the following Contracting State(s): TR)

1. A sustained release oral dosage form comprising:
(a) a wall defining a compartment, the wall comprising a semipermeable layer;
(b) an expandable layer located within the compartment and in fluid communication with the semipermeable layer;
(c) a capsule located within the compartment and in direct or indirect contacting relationship with the expandable layer, the capsule comprising a liquid antiviral drug composition comprising an antiviral drug solubilized in a solvent, which composition is substantially free of in-situ aggregation effect of the antiviral drug and provides substantially improved bioavailability of said antiviral drug; and
(d) an exit orifice formed or formable in the dosage form extending from the external surface of the capsule to the environment of use.

2. A dosage form according to claim 1 wherein the expandable layer is located within the capsule and is remote from the exit orifice.

3. A dosage form according to claim 2 further comprising a barrier layer located within the capsule between the antiviral drug composition and the expandable layer.

4. A dosage form according to claim 1 wherein the expandable layer is located within the compartment between the capsule and the semipermeable layer.

5. A dosage form according to claim 4 further comprising a barrier layer located within, the compartment between the capsule and the expandable layer.

6. A dosage form according to any preceding claim wherein said semipermeable layer comprises a semipermeable polymer; and the expandable layer comprises a hydrophilic polymer.

7. A dosage form according to claim 6 wherein the expandable layer further comprises a lubricant and/or an osmotically effective compound.

8. A dosage form according to claim 6 or 7 wherein the hydrophilic polymer is present in an amount of up to 95 wt%; the osmotically effective agent is present in an amount of 0 wt% to 60 wt%; and the lubricant is present in an amount of 0 wt% to 5 wt% of the total composition of the expandable layer.

9. A dosage form according to any preceding claim wherein said solvent comprises a surfactant, an oil or mixtures thereof.

10. A dosage form according to claim 9 wherein said surfactant is a non-ionic surfactant.

11. A dosage form according to any preceding claim wherein said liquid antiviral drug composition comprises a hydrogel and/or an osmagent.

12. A dosage form according to any one preceding claim wherein said antiviral drug is present in an amount of 5 wt% to 60 wt% and the solvent is present in an amount of 20 wt% to 95 wt% of the total antiviral drug composition.

13. A dosage form according to any preceding claim wherein the antiviral drug is selected from the group consisting of acyclovir, azidouridine, anasmycin, amantadine, bromovinyldeoxusidine, chlorovinyldeoxusidine, cytarbine, didanosine, deoxynojirmycin, dideoxycitidine, dideoxyinosine, dideoxynudeoside, desciclovir, deoxyacyclovir, edoxuidine, enviroxime, fiacitabine, foscarnet, fialuridine, .fluorothymidine, fluxuridine, ganciclovir, hypericin, interferon, interlen-kin, isethionate, idoxuridine, nevirapine, pentamidine, ribavirin, rimantadine, stavirdine, sargramostin, suramin, trichosanthin, trifluorothymidine, tribromothymidine, trichlorothymidine, vidarabine, zidoviridine, zalcitabine, 3-azido-3-deoxythymidine, saquinavir, adefovir, ritonavir, indinavir, nelfinavir, amprenavir, zidovudine and zalcitabin.

14. A dosage form according to any preceding claim wherein said antiviral drug is a protease inhibitor.

15. A dosage form according to any preceding claim wherein the capsule is a gelatin capsule.

16. A dosage form according to claim 15 which can produce an average steady-state plasma concentration of the antiviral drug greater than the therapeutically effective concentration of the antiviral drug over a period of about 4 hours to about 24 hours.

17. A dosage form according to any preceding claim for use in treating a condition in a subject responsive to the antiviral drug, wherein said condition is acquired immune deficiency syndrome (AIDS) associated with human immunodeficiency virus (HIV) infection in the subject.

18. A dosage form according to any preceding claim which can administer a therapeutically effective dose of said antiviral drug over a period of at least 4 hours after administration with no more than 30% by weight of said drug composition being released within the first 1 hour after oral administration.

19. A dosage form according to any one of claims 1 to 17 which can administer a therapeutically effective dose of said antiviral drug over a period of at least 12 hours after administration with no more than 30% by weight of said drug composition being released within the first 4 hours after oral administration.

20. A dosage form according to any one of claims 1 to 17 which can administer a therapeutically effective dose of said antiviral drug over a period of 24 hours after administration with no more than 30% by weight of said drug composition being released within the first 12 hours after oral administration.

## Patentansprüche (Patentansprüche für folgende(n) Vertragsstaat(en): AT, BE, CH, DE, DK, ES, FR, GB, GR, IE, IT, LI, LU, MC, NL, PT, SE, FI, CY)

1. Orale Dosierungsform mit verzögerter Freisetzung, umfassend:
(a) eine Wand, die eine Kammer definiert, wobei die Wand eine semi-permeable Schicht umfasst;
(b) eine expandierbare Schicht, die in der Kammer angeordnet ist und mit der semipermeablen Schicht Ober eine Flüssigkeit in Verbindung steht;
(c) eine Kapsel, die in der Kammer angeordnet ist und in direkter oder Indirekter Kontaktbeziehung mit der expandierbaren Schicht steht, wobei die Kapsel eine flüssige, antivirale Medikamentenzusammensetzung enthält, die ein antivirales Medikament umfasst, das in einem Lösungsmittel löslich gemacht ist wobei das antivirate Medikament in einer Menge von 5 Gew.-% bis 60 Gew.-%, und das Lösungsmittel in einer Menge von 20 Gew: % bis 95 Gew.-% der gesamten antiviralen Medikamentenzusammensetzung vorhanden ist, und wobei die Zusammensetzung im wesentlichen frei vom in-situ-Aggregationseffekt des antiviraien Medikaments ist und eine wesentlich verbesserte Bioverfügbarkeit dieses antiviralen Medikaments zur Verfügung stellt;
(d) eine Ausgangsöffnung, die in der Dösierungsform geformt oder formbar ist und sich von: der Außenfläche der Kapsel bis zur Einsatzumgebung erstreckt;
unter Ausschluß einer oralen Dosierung mit verzögerter Freisetzung, wie sie in diesem Patentanspruch definiert ist, worin aber:
(A) die Wand aus einer wandbildenden Zusammensetzung gebildet ist, die 85 Gew.-% Celluloseacetat mit einem Acetylgehalt von 39,8 Gew.-% und 15 Gew.-% Polyethylenglycol 3350 umfasst, wobei die Wandzusammensetzung in einem Hilfslösungsmittel aus Aceton/Methanol (80/20 Gew./Gew.) aufgelöst wurde, um eine Lösung mit 4% Feststoff herzustellen, und im Wirbelschichtbeschichter auf diese Kapsel gesprüht wurde, wobei die erhaltenen, mit einer semipermeablen Wand beschichteten Kapseln 1 Tag lang in einem Ofen bei 50 °C und 50 % relativer Luftfeuchtigkeit getrocknet wurden, um die gesamten Lösungsmittel zu entfernen; und
(B) die expandierbare Schicht aus 250 mg Granulat besteht, das zu einer tablettenförmigen Schicht in einer Presse von 0,71 cm (9/32 inch) gepresst, festgestampft und anschließend unter einer Kraft von 1 Tonne gepresst wurde, wobei das Granulat hergestellt wurde durch (i) Sprühen der fluidisierten Pulver aus 30 Gew.-% Natriumchlorid, die durch ein 20 mesh-Sieb gesiebt wurden, 58,75 Gew.-% Polyethylenoxid mit einer Molmasse von 2 000 000, 5 Gew.-% Hydroxypropylmethylcellulose mit einer Molmasse von 9200 und 1 Gew.-% rotem Eisenoxid, mit 5 Gew.-% Hydroxypropylcellulose, gelöst in gereinigtem Wasser, und (ii) Vermischen des in (i) erhaltenen Granulats mit 0,25 Gew.-% Magnesiumstearat, und
(C) die Kapsel eine flüssige, antivirale Medikamentenzusammensetzung umfasst, die aus einer homogenen Mischung aus 50 Gew.-% microfluidisiertem Acyclovir, 12,5 Gew.-% Polyoxyethylen-20-stearat, 25 Gew.-% Polyoxyl-35-kastoröl und 12, 5 Gew.-% Polyoxyl-40-stearat besteht, wobei der Kapselkörper zuerst mit der flüssigen, antiviralen Medikamentenzusammensetzung, dann mit einem Kolben aus Polyethylen hoher Dichte und anschließend mit der eXpandierbaren Schicht versehen wurde, und der gefüllte Kapselkörper mit einem Gelatinedeckel geschlossen wurde; und
(D) die Ausgangsöffnung in die Wand gebohrt wurde.

2. Dosierungsform nach Anspruch 1, worin die expandierbare Schicht in der Kapsel und von der Ausgangsöffnung entfernt angeordnet ist.

3. Dosierungsform nach Anspruch 2, die weiterhin eine Barriereschicht umfasst, die in der Kapsel zwischen der antiviralen Medikamentenzusammensetzung und der expandierbaren Schicht angeordnet ist.

4. Dosierungsform nach Anspruch 1, worin die expandierbare Schicht in der Kammer zwischen der Kapsel und der semipermeabien Schicht angeordnet ist.

5. Dosierungsform nach Anspruch 4, die weiterhin eine Barriereschicht umfasst, die in der Kammer zwischen der Kapsel und der expandierbaren Schicht angeordnet ist.

6. Dosierungsform nach einem der vorhergehenden Ansprüche, worin die semi-permeable Schicht ein semipermeables Polymer umfasst, und die expandierbare Schicht ein hydrophiles Polymer umfasst.

7. Dosierungsform nach Anspruch 6, worin die expandierbare Schicht weiterhin ein Gleitmittel und/oder eine osmotisch wirksame Verbindung umfasst.

8. Dosierungsform nach Anspruch:6 oder 7, worin das hydrophile Polymer in einer Menge von bis zu 95 Gew.-% vorliegt, das osmotisch wirksame Mittel in einer Menge von 0 Gew.-% bis 60 Gew.-% vorliegt, und das Gleitmittel in einer Menge von 0 Gew.-% bis 5 Gew.-% der Gesamtzusammensetzung der expandierbaren Schicht vorliegt.

9. Dosierungsform nach einem der vorhergehenden Ansprüche, worin das Lösungsmittel ein Tensid, ein Öl oder Gemische davon umfasst.

10. Dosierungsform nach Anspruch 9, worin das Tensid ein nicht-ionisches Tensid ist.

11. Dosierungsform nach einem der vorhergehenden Ansprüche, worin die flüssige, antivirale Medikamentenzusammensetzung ein Hydrogel und/oder ein ösmotisches Mittel umfasst.

12. Dosienrngsform nach einem der vorhergehenden Ansprüche, worin das antivirale Medikament ausgewählt ist aus der Gruppe, die aus Acyclovir, Azidouridin, Anasmycin, Amantadin, Bromvinyideoxusidin, Chlorvinyideoxusidin, Cytarbin, Didanosin, Desoxynojirmycin, Didesoxytidin, Didesoxyinosin, Didesoxynudeosid, Desciclovir, Desoxyacyclovir, Edoxuidin, Enviro)dm,- Fiacitabin, Foscamet, Fialuridin, Fluorthymidin, Fluxuridin, Ganciclovir, Hypericin, interferon, Interienkin, Isethionat, Idoxuridin, Nevirapin, Pentamidin, Ribavirin, Rimantadin, Stavirdin, Sargramostin, Suramin; Trichosanthin, Trifluorthymidin, Tribromthymidin, Trichlorthymidin, Vidarabin, Zidoviridln, Zalcitabin, 3-Azido-3-desoxythymidih, Saquinavir, Adefovir, Ritonavir, Indinavir, Nelfinavir, Amprenavir, Zidovudin und Zalcitabin besteht.

13. Dosierungsform nach einem der vorhergehenden Ansprüche, worin das antivirate Medikament ein Proteaseinhibitor ist

14. Dosierungsfonn nach einem der vorhergehenden Maprüche, worin die Kapsel eine Gelatinekapsel ist.

15. Dosierungsform nach Anspruch 14, die eine mittlere Pläsmagleichgewichtskonzentration des antiviralen Medikaments erzeugen kann, diel größer ist als die therapeutisch effektive Konzentration des antiviralen Medikaments Ober einen Zeitraum von etwa 4 Stunden bis 24 Stunden.

16. Dosierungsform nach einem der vorhergehenden Ansprüche zur Verwendung bei der Behandlung eines Krankheitszuständs bei einem Patienten, der auf das antivirale Medikament anspricht, Wobei der Krankheitszustand erworbenes Immundefektsyndrom (AIDS / acquired immune deficiency syndrom) zusammen mit HIV (human immunodeficiency virus)-infektion im Patienten ist.

17. Dosierungsform nach einem der vorhergehenden Ansprüche, die eine therapeutisch wirksame Dosis des antiviralen Medikaments über einen Zeitraum von mindestens 4 Stunden nach Verabreichung verabreichen kann, wobei nicht mehr als 30 Gew.-% der Medikamentenzusammensetzung innerhalb der ersten Stunde nach oraler Verabreichung freigesetzt werden.

18. Dosierungsform nach einem der Ansprüche 1 bis 16, die eine therapeutisch wirksame Dosis des antiviralen Medikaments Ober einen Zeitraum von mindestens 12 Stunden nach Verbreichung verabreichen kann, wobei nicht mehrials 30 Gew.-% der Medikamentenzusammensetzung innerhalb der ersten 4 Stunden nach oraler Verabreichung freigesetzt werden.

19. Dosierungsform nach einem der Ansprüche 1 bis 16, die eine therapeutisch wirksame Dosis des antiviralen Medikaments Ober einen Zeitraum von 24 Stunden nach Verabreichung verabreichen kann, wobei nicht mehr als 30 Gew.-% der Medikamentenzusamrnensetzung innerhalb der ersten 12 Stunden nach oraler Verabreichung freigesetzt werden.

## Patentansprüche (Patentansprüche für folgende(n) Vertragsstaat(en): TR)

1. Orale Dosierungsform mit verzögerter Freisetzung, umfassend:
(a) eine Wand, die eine Kammer definiert, wobei die Wand eine semi-permeable Schicht umfasst;
(b) eine expandierbare Schicht, die in der Kammer angeordnet ist und mit der semipermeablen Schicht über eine Flüssigkeit in Verbindung steht;
(c) eine Kapsel, die in der Kammer angeordnet ist und in direkter oder indirekter Kontaktbeziehung mit der expandierbaren Schicht steht, wobei die Kapsel eine flüssige, antivirale Medikamentenzusammensetzung enthält, die ein antivirales Medikament umfasst, das in einem Lösungsmittel löslich gemacht ist, und wobei die Zusammensetzung im wesentlichen frei ist vom *in-situ-*Aggregationseffekt des antiviralen Medikaments und eine wesentlich verbesserte Bioverfugbarkeit des antiviralen Medikaments zur Verfügung stellt;
und
(d) eine Ausgangsöffnung, die in der Dosierungsform geformt oder formbar ist und sich von der Außenfläche der Kapsel bis zur Einsatzumgebung erstreckt.

2. Dosierungsform nach Anspruch 1, worin die expandierbare Schicht in der Kapsel und von der Ausgangsöffnung entfernt angeordnet ist.

3. Dosierungsform nach Anspruch 2, die weiterhin eine Barriereschicht umfasst, die in der Kapsel zwischen der antiviralen Medikamentenzusammensetzung und der expandierbaren Schicht angeordnet ist.

4. Dosierungsform nach Anspruch 1, worin die expandierbare Schicht in der Kammer zwischen der Kapsel und der semipermeablen Schicht angeordnet ist

5. Dosierungsform nach Anspruch 4, die weiterhin eine Barnereschicht umfasst, die in der Kammer zwischen der Kapsel und der expandierbaren Schicht angeordnet ist.

6. Dosierungsform nach einem der vorhergehenden Ansprüche, worin die semi-permeable Schicht ein semipermeables Polymer umfasst, und die expandierbare Schicht ein hydrophiles Polymer umfasst.

7. Dosierungsform nach Anspruch 6, worin die expandierbare Schicht weiterhin ein Gleitmittel und/oder eine osmotisch wirksame Verbindung umfasst.

8. Dosierungsform nach Anspruch 6 oder 7, worin das hydrophile Polymer in einer Menge von bis zu 95 Gew.-% vorliegt, das osmotisch wirksame Mittel in einer Menge von 0 Gew.-% bis 60 Gew.-% vorliegt, und das Gleitmittel in einer Menge von 0 Gew.-% bis 5 Gew.-% der Gesamtzusammensetzung der expandierbaren Schicht vorliegt.

9. Dosierungsform nach einem der vorhergehenden Ansprüche, worin das Lösungsmittel ein Tensid, ein Öl oder Gemische davon umfasst.

10. Dosierungsform nach Anspruch 9, worin das Tensid ein nicht-ionisches Tensid ist.

11. Dosierungsform nach einem der vorhergehenden Ansprüche, worin die flüssige, antivirale Medikamentenzusammensetzung ein Hydrogel und/oder ein osmotisches Mittel umfasst.

12. Dosierungsform nach einem der vorhergehenden Ansprüche, worin das antivirale Medikament in einer Menge von 5 Gew.-% bis 60 Gew.-%, und das Lösungsmittel in einer Menge von 20: Gew.-% bis 95 Gew.-% der gesamten antiviralen Medikamentenzusammensetzung vorliegt.

13. Dosierungsform nach einem der vorhergehenden Ansprüche, worin das antivirale Medikament ausgewählt ist aus der Gruppe, die aus Acyclovir, Azidoundin, Anasmycin, Amantadin, Bromvinyldeoxusidin, Chlorvinyldeoxusidin, Cytarbin, Didanosin, Desoxynojirmycin, Didesoxyciddin, Didesoxyinosin, Didesoxynudeosid, Desciclovir, Desoxyacyclovir, Edoxuidin, Enviroxim, Fiacitabin, Foscamet, Fiaturidin, Fluorthymidin, Fluxuridin, Ganciclovir, Hypericin, Interferon, interlenkin, Isethionat, Idoxuridin, Novirapin, Pentamidin, Ribavirin, Rimantadin, Stavirdin, Sargramostin, Suramin, Trichosanthin, trifluorthymidin, Tribromthymidin, Trichlorthymdin, Vidarabin, Zidoviridin, Zalcitabin, 3-Azido-3-desoxythymidin, Saquinavir, Adefovir, Ritonavir, Indinavir, Nelfinavir, Amprenavir, Zidovudin und Zalcitabin besteht.

14. Dosierungsfiorm nach einem der vorhergehenden Ansprüche, worin das antivirale Medikament ein Proteaseinhibüor ist

15. Dosierungsform nach einem der vorhergehenden Ansprüche, worin die Kapsel eine Gelatinekapsel ist.

16. Dosierungsform nach Anspruch 15, die eine mittlere Plasmagleichgewichtskonzentration des antiviralen Medikaments erzeugen kann, die großer ist als die therapeutisch effektive Konzentration des antiviralen Medikaments über einen Zeitraum von etwa 4 Stunden bis 24 Stunden.

17. Dosierungsform nach einem der vorhergehenden Ansprüche zur Verwendung bei der Behandlung eines Krankheitszustands bei meinem Patienten, der auf das antivirale Medikament anspricht, wobei der Krankheitszustand erworbenes immundefektsyndrom (AIDS / acquired immune deficiency syndrom) zusammen mit HIV (human immunodeficiency virus)-infektion im Patienten ist.

18. Dosierungsform nach einem der vorhergehenden Ansprüche, die eine therapeutisch wirksame Dosis des antiviralen Medikaments Ober einen Zeitraum von mindestens 4 Stunden nach Verabreichung verabreichen kann, wobei nicht mehr als 30 Gew.-% der Medikamentenzusammensetzung innerhalb der ersten Stunde nach oraler Verabreichung freigesetzt werden.

19. Doslerungsform nach einem der Ansprüche 1 bis 17, die eine therapeutisch wirksame Dosis des antiviralen Medikaments über einen Zeitraum von mindestens 12 Stunden nach Verbreichung verabreichen kann, wobei nicht mehr als 30 Gew.-% der Medikamentenzusammensetzung innerhalb der ersten 4 Stunden nach oraler Verabreichung freigesetzt werden.

20. Dosierungsfarm nach einem der Ansprüche 1 bis 17, die eine therapeutisch wirksame Dosis des antiviralen Medikaments über einen Zeitraum von 24 Stunden nach Verabreichung verabreichen kann, wobei nicht mehr als 30 Gew.-% der Medikamentenzusammensetzung innerhalb der ersten 12 Stunden nach oraler Verabreichung freigesetzt werden.

## Revendications (Revendications pour l'(les) Etat(s) contractant(s) suivant(s): AT, BE, CH, DE, DK, ES, FR, GB, GR, IE, IT, LI, LU, MC, NL, PT, SE, FI, ET CY)

1. Forme posologique orale à libération entretenue, comprenant :
(a) une paroi définissant un compartiment, la paroi comprenant une couche semi-perméable ;
(b) une couche expansible située à l'intérieur du compartiment et en communication de fluide avec la couche semi-perméable ;
(c) une capsule située à l'intérieur du compartiment et en relation de contact direct ou indirect avec la couche expansible, la capsule comprenant une composition de médicament antiviral liquide comprenant un médicament antiviral solubilisé dans un solvant, ledit médicament antiviral étant présent dans une quantité de 5% en poids à 60% en poids et le solvant étant présent dans une quantité de 20% en poids à 95% en poids de la composition de médicament antiviral totale, laquelle composition est sensiblement exempte d'un effet d'agrégation *in situ* du médicament antiviral et fournit une biodisponibilité sensiblement améliorée dudit médicament antiviral ; et
(d) un orifice de sortie formé ou formable dans la forme posologique s'étendant de la surface externe de la capsule à l'environnement d'utilisation ;
à l'exclusion d'une forme posologique à libération entretenue telle que définie dans cette revendication, mais dans laquelle :
(A) ladite paroi est formée à partir d'une composition de formation de paroi comprenant 85% en poids d'acétate de cellulose comprenant une teneur en acétyle de 39,8% en poids et 15% en poids de polyéthylène glycol 3350, ladite composition de paroi ayant été dissoute dans un mélange de solvants acétone/méthanol (80/20 poids/poids) pour former une solution à 4% de matières solides et ayant été pulvérisée sur ladite capsule dans un dispositif d'enrobage en suspension dans l'air, les capsules enrobées par la paroi semi-perméables résultantes ayant été séchées dans un four à une température de 50°C et sous une humidité relative de 50° pendant 1 jour pour éliminer tous les solvants ; et
(B) ladite couche expansible consiste en 250 mg de granulés qui ont été comprimés en une couche en forme de comprimé dans un emporte-pièce de 9/32 pouce, tassée puis comprimée sous une force de 1 tonne métrique, lesdits granulés ayant été préparés par (i) pulvérisation dans un granulateur des poudres fluidisées de 30% en poids de chlorure de sodium tamisé à travers un tamis de 20 mesh d'ouverture de maille, 58,75% en poids de poly(oxyde d'éthylène) d'une masse moléculaire de 2 000 000, 5% en poids d'hydroxypropylméthylcellulose d'une masse moléculaire de 9200 et 1% en poids d'oxyde ferrique rouge, avec 5% en poids d'hydroxypropylcellulose dissoute dans de l'eau purifiée, et (ii) mélange des granulés obtenus en (i) avec 0,25% en poids de stéarate de magnésium ; et
(C) ladite capsule comprend une composition de médicament antiviral liquide consistant en un mélange homogène de 50% en poids d'acyclovir microfluidisé, 12,5% en poids de stéarate de polyoxyéthylène 20, 25% en poids d'huile de ricin polyoxylée 35 et 12,5% en poids de stéarate polyoxylé 40, le corps de capsule ayant été chargé tout d'abord par ladite composition de médicament antiviral liquide, puis par un piston formé de polyéthylène haute densité, puis par la couche expansible, le corps de capsule rempli ayant été fermé par le capuchon de gélatine ; et
(D) ledit orifice de sortie a été foré dans ladite paroi.

2. Forme posologique seloh la revendication 1, dans laquelle la couche expansible est située à l'intérieur de la capsule et est éloignée de l'orifice de sortie.

3. Forme posologique selon la revendication 2, comprenant en outre une couche barrière située à l'intérieur de la capsule entre la composition de médicament antiviral et la couche expansible.

4. Forme posologique selon la revendication 1, dans laquelle la couche expansible est située à l'intérieur du compartiment entre la capsule et la couche semi-perméable.

5. Forme posologique selon la revendication 4, comprenant en outre une couche barrière située à l'intérieur du compartiment entre la capsule et la couche expansible.

6. Forme posologique selon l'une quelconque des revendications précédentes, dans laquelle ladite couche semi-perméable comprend un polymère semi-perméable ; et la couche expansible comprend un polymère hydrophile.

7. Forme posologique seloh la revendication 6, dans laquelle la couche expansible comprend en outre un lubrifiant et/ou un composé osmotiquement efficace.

8. Forme posologique selon l'une des revendications 6 ou 7, dans laquelle le polymère hydrophile est présent dans une quantité allant jusqu'à 95% en poids ; l'agent osmotiquement efficace est présent dans une quantité de 0% en poids: à 60% en poids ; et le lubrifiant est présent dans une quantité de 0% en poids à 5% en poids de la composition totale de la couche expansible.

9. Forme posologique selon l'une quelconque des revendications précédentes, dans laquelle lediti solvant comprend un agent tensio-actif, une huile ou des mélanges de ceux-ci.

10. Forme posologique selon la revendication 9, dans laquelle ledit agent tensio-actif est un agent tensio-actif non-ionique.

11. Forme posologique selon l'une quelconque des revendications précédentes, dans laquelle ladite composition de médicament antiviral liquide comprend un hydrogel et/ou un osmagent.

12. Forme posologique selon l'une quelconque des revendications précédentes, dans laquelle le médicament antiviral est choisi dans le groupe constitué par l'acyclovir, l'azidouridine, l'anasmycine, l'amantadine, la bromovinyldéoxusidine, la chlorovinyldéoxusidine, la cytarbine, la didanosine, la désoxynojirmycine, la didéoxycitidine, la didéoxyinosine, le didéoxynudéoside, le desciclovir, le déoxyacyclovir, l'édoxuidine, l'enviroxime, la fiacitabine, le foscarnet, la fialuridine, la fluorothymidine, la fluxuridine, le ganciclovir, l'hypéricine, l'interféron, l'interlenkine, l'iséthionate, l'idoxuridine, la névirapine, la pentamidine, la ribavirine, la rimantadine, la stavirdine, la sargramostine, la suramine, la trichosanthine, la trifluorothymidine, la tribromothymidine, la trichlorothymidine, la vidarabine, la zidoviridine, la zalcitabine, la 3-azidb-3-déoxythymidine, le saquinavir, l'adéfovir, le ritonavir, l'indinavir, le nelfinavir, l'amprénavir, la zidovudine et la zalcitabine.

13. Forme posologique selon l'une quelconque des revendications précédentes, dans laquelle ledit médicament antiviral est un inhibiteur de protéases.

14. Forme posologique selon l'une quelconque des revendications précédentes, dans laquelle la capsule est une capsule de gélatine.

15. Forme posologique seloh la revendication 14, qui peut produire une concentration moyenne dans le plasma à l'état d'équilibre du médicament antiviral supérieure à la concentration thérapeutiquement efficace du médicament antiviral sur une période d'environ 4 heures à environ 24 heures.

16. Forme posologique selon l'une quelconque des revendications précédentes destinée à être utilisée dans le traitement d'un état dans un sujet sensible au médicament antiviral, dans lequel ledit état est le syndrome immunodéficitaire acquis (SIDA) associé à une infection par le virus du syndrome immunodéficitaire acquis humain (HIV) dans le sujet.

17. Forme posologique selon l'une quelconque des revendications précédentes, qui peut administrer une dose thérapeutiquement efficace dudit médicament antiviral sur une période d'au moins 4 heures après administration, avec pas plus de 30% en poids de ladite composition de médicament qui sont libérés dans la première heure après l'administration orale.

18. Forme posologique selon l'une quelconque des revendications 1 à 16, qui peut administrer une dose thérapeutiquement efficace dudit médicament antiviral sur une période d'au moins 12 heures après administration, avec pas glus de 30% en poids de ladite composition de médicament qui sont libérés dans les 4 premières heures après l'administration orale.

19. Forme posologique selon l'une quelconque des revendications 1 à 16, qui peut administrer une dose thérapeutiquement efficace dudit médicament antiviral sur une période de 24 heures après administration, avec pas plus de 30 % en poids de ladite composition de médicament qui sont libérés dans les 12 premières heures après l'administration orale.

## Revendications (Revendications pour l'(les) Etat(s) contractant(s) suivant(s): TR)

1. Forme posologique orale à libération entretenue, comprenant :
(a) une paroi définissant un compartiment, la paroi comprenant une couche semi-perméable ;
(b) une couche expansible située à l'intérieur du compartiment et en communication de fluide avec la couche semi-perméable ;
(c) une capsule située à l'intérieur du compartiment et en relation de contact direct ou indirect avec la couche expansible, la capsule comprenant une composition de médicament antiviral liquide comprenant un médicament antiviral solubilisé dans un solvant, laquelle composition est sensiblement exempte d'effet d'agrégation in situ du médicament antiviral et fournit une biodisponibilité sensiblement améliorée dudit médicament antiviral ; et
(d) un orifice de sortie formé ou formable dans la forme posologique s'étendant de la surface externe de la capsule à l'environnement d'utilisation.

2. Forme posologique selon la revendication 1, dans laquelle la couche expansible est située à l'intérieur de la capsule et est éloignée de l'orifice de sortie.

3. Forme posologique selon la revendication 2, comprenant en outre une couche barrière située à l'intérieur de la capsule entre la composition de médicament antiviral et la couche expansible.

4. Forme posologique selon la revendication 1, dans laquelle la couche expansible est située à l'intérieur du compartiment entre la capsule et la couche semi-perméable.

5. Forme posologique selon la revendication 4, comprenant en outre une couche barrière située à l'intérieur du compartiment entre la capsule et la couche expansible.

6. Forme posologique selon l'une quelconque des revendications précédentes, dans laquelle ladite couche semi-perméable comprend un polymère semi-perméable ; et la couche expansible comprend un polymère hydrophile.

7. Forme posologique selon la revendication 6, dans laquelle la couche expansible comprend en outre un lubrifiant et/ou un composé osmotiquement efficace:.

8. Forme posologique selon l'une des revendications 6 ou 7, dans laquelle le polymère hydrophile est présent dans une quantité allant jusqu'à 95% en poids ; l'agent osmotiquement efficace est présent dans une quantité de 0% en poids à 60% en poids ; et le lubrifiant est présent dans une quantité de 0% en poids à 5% en poids de la composition totale de la couche expansible. ,

9. Forme posologique selon l'une quelconque des revendications précédentes, dans laquelle ledit: solvant comprend un agent tensio-actif, une :huile ou des mélanges de ceux-ci.

10. Forme posologique selon la revendication 9, dans laquelle ledit agent tensio-actif est un agent tensio-actif non-ionique.

11. Forme posologique selon l'une quelconque des revendications précédentes, dans laquelle ladite composition de médicament antiviral liquide comprend un hydrogel et/ou un osmagent.

12. Forme posologique seloh l'une quelconque des revendications précédentes, dans laquelle ledit médicament antiviral est présent dans une quantité de 5% en poids à 60% en poids et le solvant est présent dans une quantité de 20% en poids à 95% en poids de la composition de médicament antiviral.

13. Forme posologique selon l'une quelconque des revendications précédentes, dans laquelle ledit médicament antiviral est choisi dans le groupe constitué par l'acyclovir, l'azidouridine, l'anasmycine, l'amantadine, la bromovinyidéoxusidine, la chlorovinyidéoxusidine, la cytarbine, la didanosine, la désoxynojirmycine, la didéoxycitidine, la didéoxyinosine, le didéoxynudéoside, le desciclovir, le déoxyacyclovir, l'édoxuidine, l'enviroxime, la fiacitabine, le foscarnet, la fialuridine, la fluorothymidine, la fluxuridine, le ganciclovir, l'hypéricine, l'interféron, l'interlenkine, l'iséthionate, l'idoxuridine, la névirapine, la pentamidine, la ribavirine, la rimantadine, la stavirdine, la sargramostine, la suramine, la trichosanthine, la trifluorothymidine, la tribromothymidine, la trichlorothymidine, la vidarabine, la zidoviridine, la zalcitabine, la 3-azido-3-déoxythymidine, le saquinavir, l'adéfovir, le ritonavir, l'indinavir, le nelfinavir, l'amprénavir, la zidovudine et la zalcitabine.

14. Forme posologique selon l'une quelconque des revendications précédentes, dans laquelle ledit médicament antiviral est un inhibiteur de protéases.

15. Forme posologique selon l'une quelconque des revendications précédentes, dans laquelle la capsule est une capsule de gélatine.

16. Forme posologique selon la revendication 15, qui peut produire une concentration moyenne dans le plasma à l'état d'équilibre du médicament antiviral supérieure à la concentration thérapeutiquement efficace du médicament antiviral sur une période d'environ 4 heures à environ 24 heures.

17. Forme posologique selon l'une quelconque des revendications précédentes destinée à être utilisée dans le traitement: d'un état dans un sujet sensible au médicament antiviral, dans lequel ledit état est le syndrome immunodéficitaire acquis (SIDA) associé à une infection par le virus du syndrome immunodéficitaire acquis humain (HIV) dans le sujet.

18. Forme posologique selon l'une quelconque des revendications précédentes, qui peut administrer une dose thérapeutiquement efficace dudit médicament antiviral sur une période d'au moins 4 heures après administration, avec pas plus de 30% en poids de ladite composition de médicament qui sont libérés dans la première heure après l'administration orale.

19. Forme posologique selon l'une quelconque des revendications 1 à 17, qui peut administrer une dose thérapeutiquement efficace dudit médicament antiviral sur une période d'au moins 12 heures après administration, avec pas plus de 30% en poids de ladite composition de médicament qui sont libérés dans les 4 premières heures après l'administration orale.

20. Forme posologique seloh l'une quelconque des revendications 1 à 17, qui peut administrer une dose thérapeutiquement efficace dudit médicament antiviral sur une période de 24 heures après administration, avec pas plus de 30% en poids de ladite composition de médicament qui sont libérés dans les 12 premières heures après l'administration orale.
